# EUROPEAN PATENT APPLICATION

(11) **EP 4 564 262 A1**
(43) Date of publication of application: **04.06.2025**
(21) Application number: 22965067.6
(22) Date of filing: 07.11.2022
(51) Int. Cl.: G06Q 10/20

(54) **MANAGEMENT DEVICE, MANAGEMENT METHOD, AND ACIDIC GAS ADSORPTION SYSTEM**

(71) Applicant: NGK Insulators, Ltd., Nagoya city, Aichi 467-8530 (JP)
(72) Inventor: OKUMA, Yusuke, Nagoya-city, Aichi 467-8530 (JP); KAN, Hirofumi, Nagoya-city, Aichi 467-8530 (JP); TAKAHASHI, Michio, Nagoya-city, Aichi 467-8530 (JP); ANDO, Junichi, Nagoya-city, Aichi 467-8530 (JP)
(74) Representative: MERH-IP Matias Erny Reichl Hoffmann Patentanwälte PartG mbB
(86) International application number: PCT/JP2022/041451
(87) International publication number: WO 2024/100740

(57) **Abstract**

Provided is a management apparatus 30 including: a concentration acquisition unit 31 configured to acquire a first concentration which is a concentration of an acidic gas when a gas containing the acidic gas is introduced into an acidic gas adsorber that adsorbs the acidic gas and a second concentration which is a concentration of the acidic gas after being processed in the acidic gas adsorber; a captured amount calculation unit 32 configured to calculate a captured amount of the acidic gas adsorbed by the acidic gas adsorber based on the first concentration and the second concentration; and a management unit 33 configured to conduct management to replace the acidic gas adsorber based on a regeneration timing of the acidic gas adsorber determined based on the calculated captured amount. The present invention thereby provides a management apparatus and a management method that facilitate the management of an acidic gas adsorber and save costs when the acidic gas adsorber is used to separate and capture an acidic gas, and an acidic gas adsorption system.

## Description

### [Technical Field]

The present invention relates to a management apparatus, a management method, and an acidic gas adsorption system. In particular, the present invention relates to a management apparatus for managing an acidic gas adsorber that adsorbs acidic gas, or the like.

### [Background Art]

In recent years, efforts have been made to separate and capture acid gases contained in the atmosphere in order to reduce the environmental load. As such an acidic gas, carbon dioxide (CO₂), which causes global warming, is mainly mentioned. Then, in order to separate and capture acidic gases, such as carbon dioxide, an acidic gas adsorber that adsorbs acidic gases may be used.

PTL 1 discloses an absorbent structure for capturing CO₂. This absorbent structure includes a honeycomb substrate having a plurality of bulkheads extending in the axis direction from the inlet end to the outlet end, which forms a plurality of channels. The honeycomb substrate includes a solidified powdery component and a binder. Furthermore, the absorbent structure includes functional structure unit groups dispersed throughout the powder component of the honeycomb substrate bulkhead. The functional structure unit groups are located in or on the bulkheads such that when a gas stream containing CO₂ flows through the channels from the inlet end to the outlet end, the functional structure unit groups form coordinate bonds with CO₂ to form carbonate salts, bicarbonate salts, a carbamate, or other coordinates, or an ionic compound, thereby absorbing CO₂.

### [Citation List]

### [Patent Literature]

[PTL 1] WO 2013/119929

### [Summary of Invention]

### [Technical Problem]

When an acidic gas adsorber is used to separate and capture an acidic gas, a used acidic gas adsorber may be regenerated and reused to save cost in some cases.

However, when a capturer of the acidic gas manages the regeneration timing, the replacement cost tends to be high because replacement person-hours for reuse increase. Furthermore, the management cost for monitoring the operation period of the acidic gas adsorber tends to be high.

The present invention has an object to provide a management apparatus and a management method that facilitate the management of an acidic gas adsorber and save costs when the acidic gas adsorber is used to separate and capture an acidic gas, and an acidic gas adsorption system.

### [Solution to Problem]

In order to solve the above problem, the present invention provides a management apparatus including: a concentration acquisition unit configured to acquire a first concentration which is a concentration of an acidic gas when a gas containing the acidic gas is introduced into an acidic gas adsorber that adsorbs the acidic gas and a second concentration which is a concentration of the acidic gas after being processed in the acidic gas adsorber; a captured amount calculation unit configured to calculate a captured amount of the acidic gas adsorbed by the acidic gas adsorber based on the first concentration and the second concentration; and a management unit configured to conduct the management to replace and regenerate the acidic gas adsorber based on a regeneration timing of the acidic gas adsorber determined based on the calculated captured amount.

The present invention also provides a management method including: acquiring a first concentration which is a concentration of the acidic gas when a gas containing the acidic gas is introduced into an acidic gas adsorber that adsorbs the acidic gas and a second concentration which is a concentration of the acidic gas after being processed in the acidic gas adsorber; calculating a captured amount of the acidic gas adsorbed by the acidic gas adsorber based on the first concentration and the second concentration; and conducting management to replace and regenerate the acidic gas adsorber based on a regeneration timing of the acidic gas adsorber determined based on the calculated captured amount.

The present invention further provides an acidic gas adsorption system including: an acidic gas adsorber configured to adsorb an acidic gas; concentration measurement means configured to measure a first concentration which is a concentration of the acidic gas when the acidic gas is introduced into the acidic gas adsorber and a second concentration which is a concentration of the acidic gas after being processed in the acidic gas adsorber; and a management apparatus configured to manage the acidic gas adsorber based on the first concentration and the second concentration; the management apparatus including: a concentration acquisition unit configured to acquire the first concentration and the second concentration from the concentration measurement means; a captured amount calculation unit configured to calculate a captured amount of the acidic gas adsorbed by the acidic gas adsorber based on the first concentration and the second concentration; and a management unit configured to conduct management to replace and regenerate the acidic gas adsorber based on a regeneration timing of the acidic gas adsorber determined based on the calculated captured amount.

### [Advantageous Effects of the Invention]

The present invention can provide a management apparatus and a management method that facilitate the management of an acidic gas adsorber and save cost when the acidic gas adsorber is used to separate and capture an acidic gas, and an acidic gas adsorption system.

### [Brief Description of Drawings]

[Fig. 1]

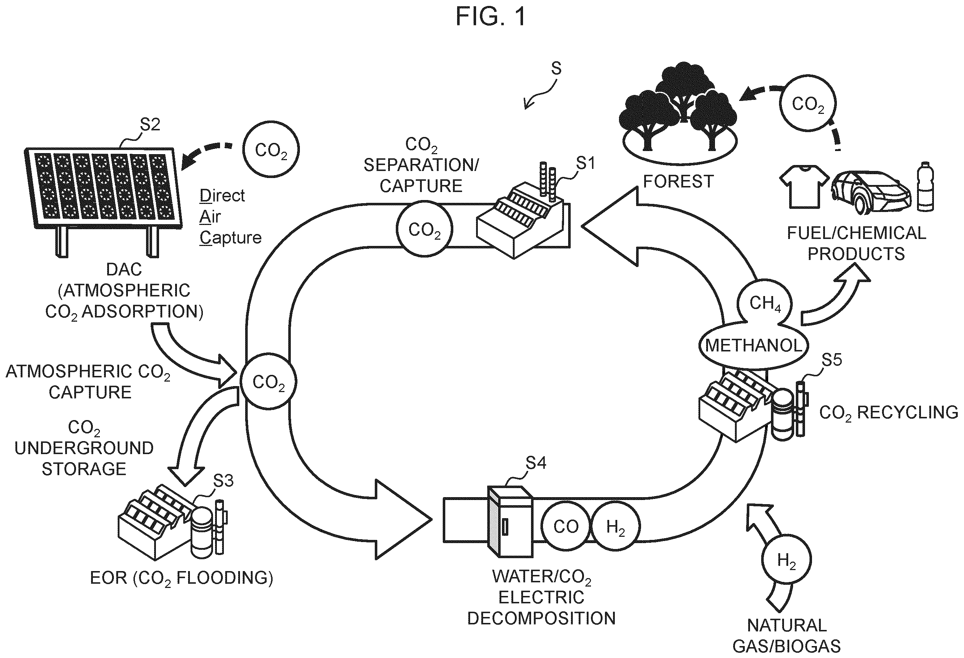

   Fig. 1 is a diagram illustrating a CCUS cycle.
[Fig. 2]

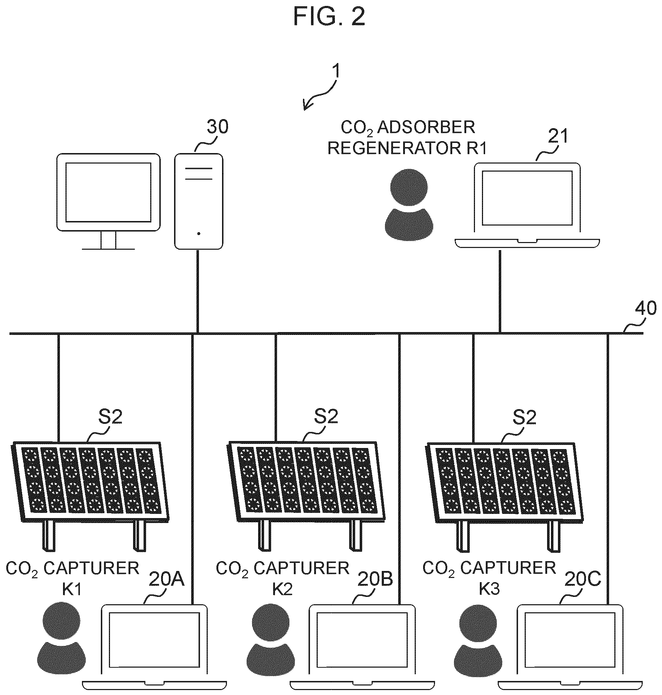

   Fig. 2 is a diagram of a CO₂ adsorption system for operating and managing a DAC system.
[Fig. 3]

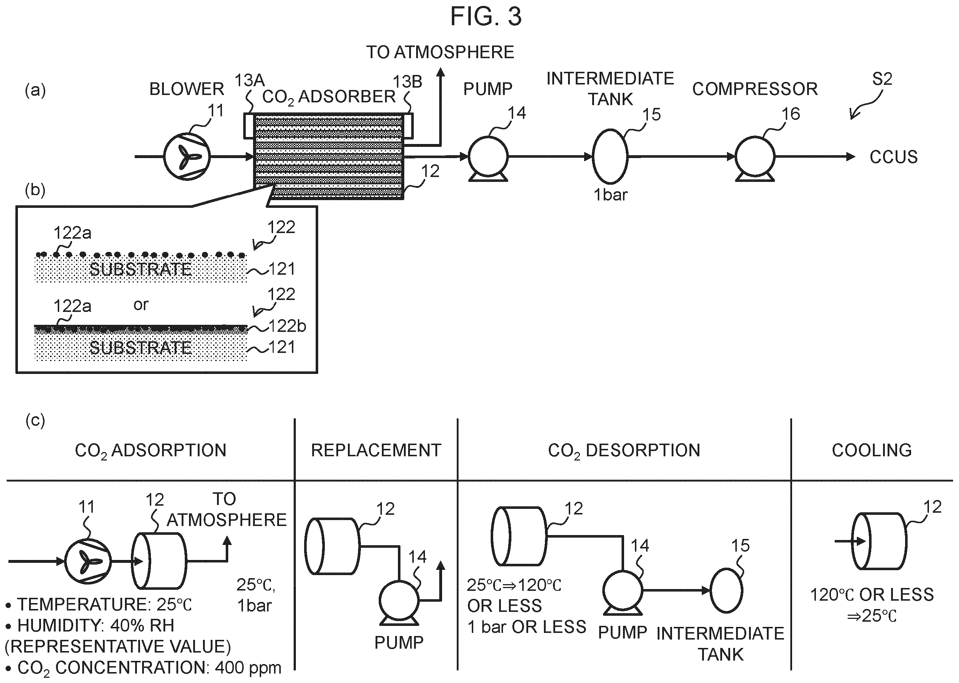

   Fig. 3(a) is a diagram illustrating the configuration of a DAC system. Fig. 3(b) is a cross-sectional view of a CO₂ adsorber, which illustrates the structure of the CO₂ adsorber. Fig. 3(c) is a diagram illustrating the operation of a DAC system.
[Fig. 4]

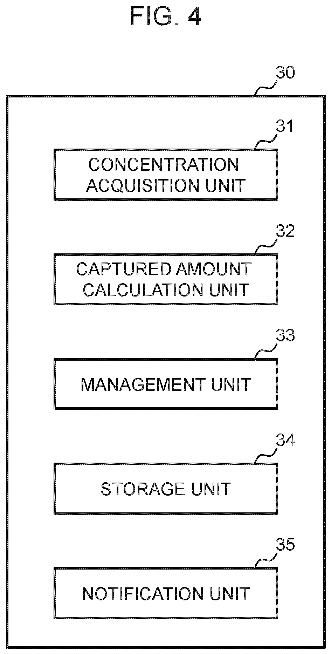

   Fig. 4 is a block diagram illustrating the functional configuration of a management apparatus.
[Fig. 5]

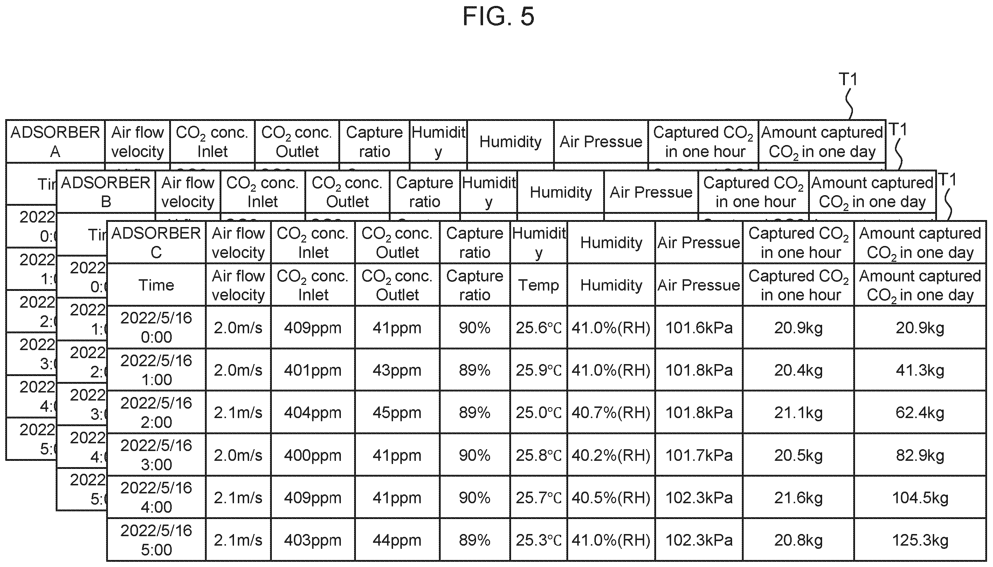

   Fig. 5 is a diagram illustrating a data structure when data are stored in the storage unit.
[Fig. 6]

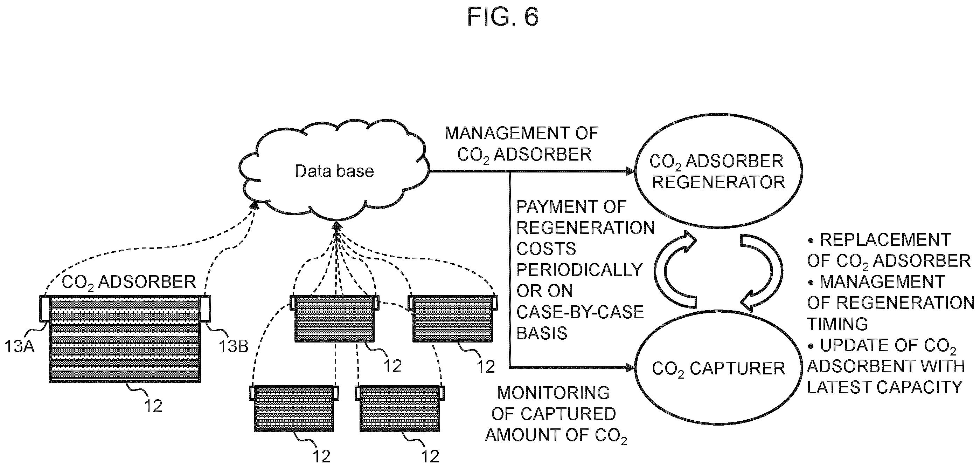

   Fig. 6 is a conceptual diagram illustrating the management method of a CO₂ adsorber.
[Fig. 7]

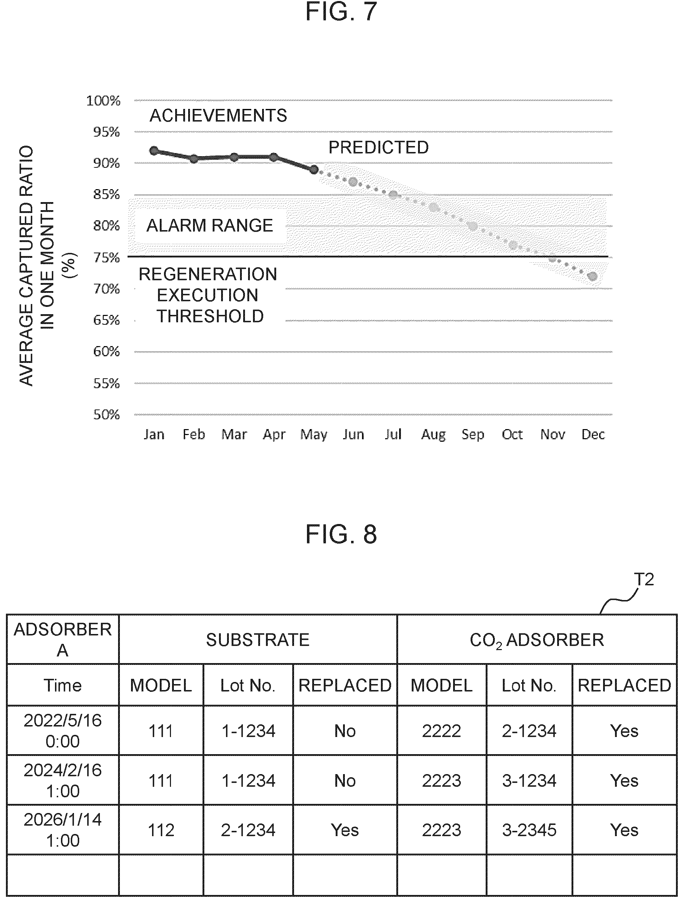

   Fig. 7 is a diagram illustrating the regeneration timing of a CO₂ adsorber.
[Fig. 8]
   Fig. 8 is a diagram illustrating a table for representing the regeneration history of a CO₂ adsorber.
[Fig. 9]

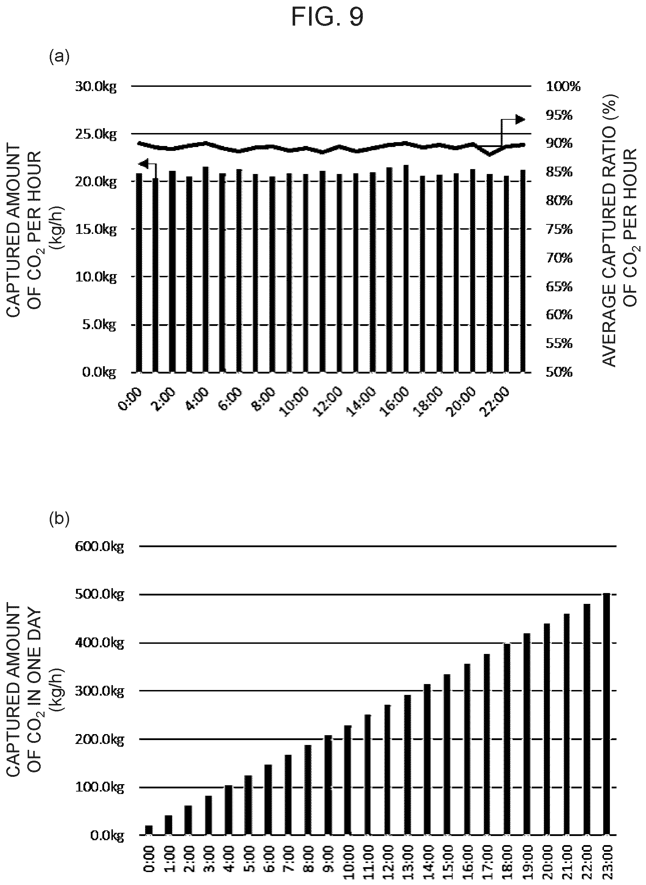

   Figs. 9(a) and 9(b) are diagrams illustrating examples of periodic reports.
[Fig. 10]

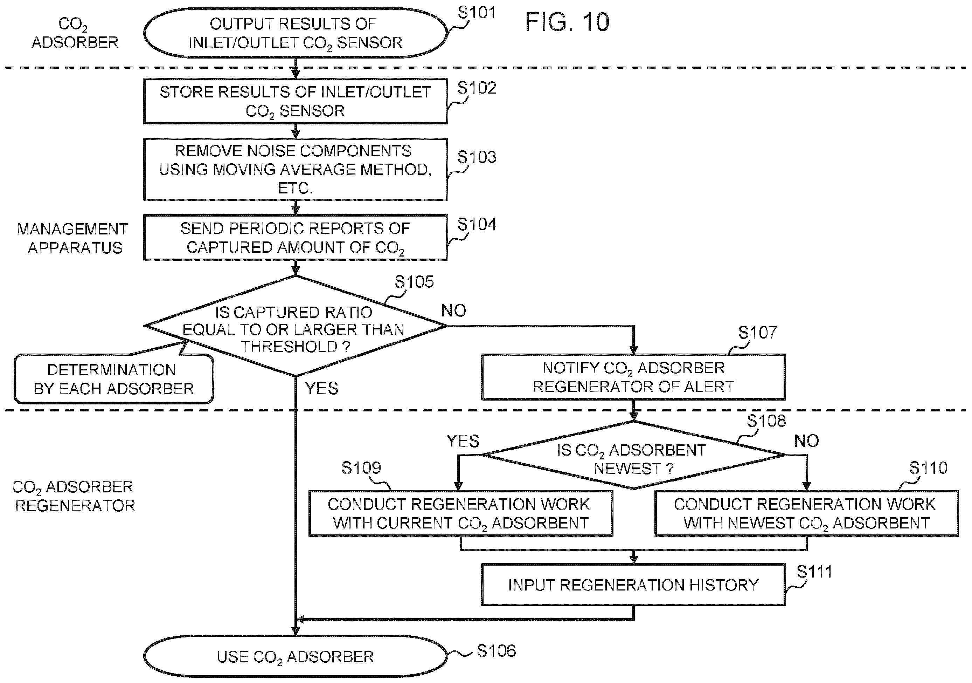

   Fig. 10 is a flowchart illustrating the operation of a CO₂ adsorption system.

### [Description of Embodiments]

Embodiments of the present invention are described below in detail with reference to the appended drawings.

As a typical example of the effort for separating or capturing carbon dioxide, the carbon dioxide capture, utilization and storage (CCUS) cycle has been known. In the CCUS cycle, the separated and captured carbon dioxide is reused to achieve a carbon-neutral society through the recycling of carbon dioxide.

### <Explanation on CCUS Cycle>

Fig. 1 is a diagram illustrating a CCUS cycle S.

The CCUS cycle S illustrated separates and captures carbon dioxide emitted from a waste disposal site or a thermal power plant S1 with a CO₂ separation membrane (CO₂ separation/capture).

A direct air capture (DAC) system S2 adsorbs carbon dioxide present in the air (Atmospheric CO₂ capture).

Captured carbon dioxide is stored, for example, under the ground (CO₂ underground storage). For example, enhanced oil recovery (EOR) is used in an oil well S3 to recover crude oil. That is, carbon dioxide is injected underground, which flushes the crude oil towards the oil well S3. This method is also CO₂ flooding. In this method, carbon dioxide injected is removed from the ground, together with the crude oil, which is then the crude oil and carbon dioxide are separated using a carbon dioxide (CO₂) separation membrane. Separated carbon dioxide is used for being injected underground again.

Captured carbon dioxide is supplied with water to, for example, a solid oxide electrolysis cell (SOEC) S4 to produce carbon monoxide (CO) and hydrogen (H₂) by electric decomposition (water/CO₂ electric decomposition). Then, using this hydrogen, or hydrogen included in natural gas or biogas, carbon dioxide and hydrogen are synthesized (methanation, methanol synthesis) in a methanation plant or methanol production plant S5 to produce methane (CH₄) or methanol (CH₃OH) (CO₂ recycling). Produced methane or methanol is used as a raw material for fuel or chemical products. The waste is incinerated, for example, at the waste disposal site or thermal power plant S1, where the carbon dioxide emitted can be recycled in the CCUS cycle S. When the Produced methane or methanol is used as fuel, it is adsorbed in the forest or a DAC system S2, for example.

### <Explanation on CO₂ Adsorbing System 1>

Fig. 2 is a diagram of a CO₂ adsorption system 1 for operating and managing the DAC system S2.

The CO₂ adsorption system 1 is one example of an acidic gas adsorption system. In the CO₂ adsorption system 1, three DAC systems S2, terminal apparatuses 20A, 20B, and 20C owned by CO₂ capturers K1, K2, and K3 who operate the DAC systems S2, respectively, a terminal apparatus 21 owned by a CO₂ adsorber regenerator R1 who regenerates CO₂ adsorbers 12 of the DAC systems S2 (see Fig. 3), and a management apparatus 30 that manages the entire CO₂ adsorption system 1 are connected via a network 40. In this embodiment, the CO₂ capturers K1, K2, and K3 are examples of acidic gas capturers. The CO₂ adsorber regenerator R1 is one example of an acidic gas adsorber regenerator. In this embodiment, the CO₂ capturers K1, K2, and K3 are three persons, and the corresponding DAC systems S2 and terminal apparatuses 20A, 20B, and 20C are also three each. Nevertheless, the number of these are any as long as the number is 1 or more. It is noted that one CO₂ capturer may operate a plurality of DAC systems S2. The number of the CO₂ adsorber regenerator R1 is one in this embodiment, but may be plural. Hereinafter, when the CO₂ capturers K1, K2, and K3 are not distinguished from one another, they each may simply be referred to as a "CO₂ capturer", and CO₂ adsorber regenerator R1 may simply be referred to as a "CO₂ adsorber regenerator". In addition, when the terminal apparatuses 20A, 20B, are 20C are not distinguished from one another, they each may simply be referred to as a "terminal apparatus 20".

The terminal apparatus 20 is an apparatus for a CO₂ capturer to monitor the operation state of the DAC system S2. The terminal apparatus 20 displays a periodic report for notifying the captured amount of carbon dioxide, as described in more detail below. This periodic report is created by the management apparatus 30 and sent to the terminal apparatus 20. This periodic report enables a CO₂ capturer to monitor the captured amount of carbon dioxide captured at the DAC system S2.

The terminal apparatus 21 is an apparatus used by a CO₂ adsorber regenerator to recognize the regeneration timing of the CO₂ adsorber 12. The terminal apparatus 21 displays a notification from the management apparatus 30 about the regeneration timing, as described in more detail below.

The terminal apparatuses 20 and 21 are, for example, computer apparatuses such as general-purpose personal computers (PC), mobile computers, mobile phones, smartphones, or tablets. Then, the terminal apparatus 20 and 21 operate various types of application software under the management of an operating system (OS).

The management apparatus 30 is a server computer that manages the entire CO₂ adsorption system 1, as described in more detail below.

It should be noted that the number of the management apparatus 30 illustrated is one, but the function may be implemented by a plurality of server computers.

The terminal apparatuses 20 and 21, and a management apparatus 30 are provided with a processor, such as a central processing unit (CPU), which is arithmetic means, and a main memory, which is a storage means. Here, the processor executes various types of software, such as operating systems (OS) or applications (application software). A main memory is a storage area for storing various types of software, data used for executing the software, and the like. Furthermore, the terminal apparatuses 20 and 21, and the management apparatus 30 are provided with a communication interface (hereinafter expressed as "communication I/F") for conducting communication with the outside; a display mechanism composed of a video memory, a display, or the like; and an input mechanism such as an input button, a touch panel, and a keyboard. Furthermore, the terminal apparatuses 20 and 21, and the management apparatus 30 are provided with a storage as an auxiliary storage apparatus. The storage is, for example, a hard disk drive (HDD) or a solid-state drive (SSD).

The network 40 is communication means used for the information communication between the DAC system S2, the terminal apparatuses 20 and 21, and the management apparatus 30, and, for example, is the Internet, a local area network (LAN), or a wide area network (WAN). The communication lines used for information communication may be either wired or wireless, and a combination of such communication lines may also be used. Furthermore, the DAC system S2, the terminal apparatuses 20 and 21, and the management apparatus 30 may be connected via a plurality of networks or communication lines using relay apparatuses such as gateway apparatuses or routers.

### <Explanation on DAC System S2>

Next, the DAC system S2 will be described in detail.

Fig. 3(a) is a diagram illustrating the configuration of the DAC system S2.

The DAC system S2 is provided with a blower 11 that sends the air; a CO₂ adsorber 12 that adsorbs carbon dioxide; CO₂ concentration sensors 13A and 13B that measure the concentration of carbon dioxide; a pump 14 that sends carbon dioxide; an intermediate tank 15 that stores carbon dioxide; and a compressor 16 that liquidizes carbon dioxide. However, depending on the type of the DAC system S2, any or all of the pump 14, the intermediate tank 15, and the compressor 16 may not be applied.

The blower 11 is an apparatus for feeding the air, which is one example of gases, to the CO₂ adsorber 12. The type of the blower 11 is not particularly limited. For example, a general fan that is provided with a plurality of blade plates (propellers) and an impeller that rotates around a main shaft and blows the air by transmitting the rotation driving force of an electric motor, such as a motor, to the impeller through the main shaft. As the blower 11, there are various types of blowers, such as sirocco fans, turbo fans, ventilation fans, and line fans, which may be applied depending on the type of impeller.

The CO₂ adsorber 12 is an example of an acidic gas adsorber and is an apparatus that adsorbs carbon dioxide in the air sent from the blower 11.

The CO₂ adsorber 12 has, for example, a filter structure such as a honeycomb or a filter cloth, a pellet structure, and an alkali solution structure. Then, the CO₂ adsorber 12 adsorbs carbon dioxide when the air transmits through the CO₂ adsorber 12.

Fig. 3(b) is a cross-sectional view of the CO₂ adsorber 12, which illustrates the structure of the CO₂ adsorber 12.

As illustrated in Fig. 3 (b), the CO₂ adsorber 12 is provided with a substrate 121 and a CO₂ adsorption layer 122 formed on the surface of the substrate 121. The CO₂ adsorption layer 122 contains a CO₂ adsorbent 122a that adsorbs carbon dioxide. The CO₂ adsorption layer 122 is one example of an acidic gas adsorption layer. The CO₂ adsorbent 122a is also one example of an acidic gas adsorbent. In this case, the CO₂ adsorption layer 122 may be composed only of the CO₂ adsorbent 122a, as illustrated on the upper side in Fig. 3(b), or may be formed so that the CO₂ adsorbent 122a is dispersed in a carrier 122b, as illustrated on the lower side of Fig. 3(b).

Typical examples of the substrate 121 may include ceramics. Examples of ceramics may include silicon carbide, silicon-silicon carbide-based composite material, cordierite, mullite, alumina, silicon nitride, spinel, silicon carbide-cordierite-based composite material, lithium aluminum silicate, and aluminum titanate. Constituent materials may be used alone or in combination.

Examples of the CO₂ adsorbent 122a may include nitrogen-containing compounds; alkali compounds such as sodium hydroxide and potassium hydroxide; carbonate salts such as calcium carbonate and potassium carbonate; hydrogencarbonate salts such as calcium hydrogencarbonate and potassium hydrogencarbonate: organic metal structures (MOF) such as MOF-74, MOF-200, and MOF-210; zeolite; activated carbon; nitrogen-doped carbon; ion liquids; and the like. Carbon dioxide adsorbents may be used alone or in combination.

However, the CO₂ adsorbent 122a is preferably a nitrogen-containing compound. More specifically, examples of nitrogen-containing compounds may include primary amines such as monoethanolamine and polyvinylamine; secondary amines such as diethanolamine, cyclic amines, and N-(3-aminopropyl)diethanolamine; tertiary amines such as methyldiethylamine and triethanolamine; ethyleneamine compounds such as tetraethylenepentamine; aminosilane coupling agents such as aminopropyltrimethoxysilane, 3-aminopropyltriethoxysilane, N-(2-aminoethyl)-3-aminopropyltrimethoxysilane, and polyethyleneimine-trimethoxysilane; imine compounds such as ethyleneimine, linear polyethyleneimine, and branched polyethylene imine having primary, secondary, or tertiary amino groups; piperazine compounds such as 1-(2-hydroxyethyl)piperazine; amide compounds such as polyamideamine; polyvinylamine; and organic/inorganic compounds with an amino group as a substituent.

For the CO₂ adsorbent 122a, the materials described above may be used alone or in combination. The mode of adsorption may be physical adsorption with active carbon or the like or chemical adsorption with a nitrogen-containing compound or the like.

The carrier 122b is, for example, a porous carrier, and examples thereof may include organic metal structures (MOF) such as MOF-74, MOF-200, and MOF-210; activated carbon; nitrogen-doped carbon; mesoporous silica; mesoporous aluminum; zeolite; carbon nanotubes; and fluororesins such as polyvinylidene fluoride (PVDF). Porous carriers may be used alone or in combination. The porous carrier is preferably a material different from the CO₂ adsorbent 122a.

The CO₂ adsorption layer 122 illustrated in Fig. 3(b) is formed on one of two principal surfaces of the substrate 121 but may be formed on the other surface. That is, the CO₂ adsorption layer 122 may be formed on both surfaces of the substrate 121.

The CO₂ concentration sensors 13A and 13B are examples of concentration measurement means and are sensors for measuring the concentration of carbon dioxide in the air. Among these, the CO₂ concentration sensor 13A is disposed on the inlet side of the CO₂ adsorber 12, where the air flows in. Accordingly, it can be said that the CO₂ concentration sensor 13A measures a first concentration, which is the concentration of carbon dioxide when a gas containing carbon dioxide is introduced into the CO₂ adsorber 12. In contrast, the CO₂ concentration sensor 13B is disposed on the outlet side of the CO₂ adsorber 12, where the air flows out. Accordingly, it can be said that the CO₂ concentration sensor 13B measures a second concentration, which is the concentration of carbon dioxide after being processed in the CO₂ adsorber 12.

The CO₂ concentration sensors 13A and 13B are not particularly limited as long as they are sensors that can measure the concentration of carbon dioxide. For example, as the CO₂ concentration sensors 13A and 13B, non-dispersive infrared (NDIR) concentration sensors may be used. That is, carbon dioxide absorbs an infrared ray at a wavelength of 4.26 µm, so the amount of infrared rays transmitted through the air is attenuated depending on the concentration of carbon dioxide in the air. Accordingly, the concentration of carbon dioxide can be measured by measuring the intensity of the infrared rays transmitted through the air at a predetermined distance from a lamp that emits infrared rays with this wavelength.

The air processed in the CO₂ adsorber 12 is released into the air. When carbon dioxide adsorbed by the CO₂ adsorber 12 is desorbed, the desorbed carbon dioxide is sent to a pump 14.

The pump 14 is disposed to send desorbed carbon dioxide to the intermediate tank 15 when the carbon dioxide absorbed by the CO₂ adsorber 12 is desorbed. The pump 14 is not particularly limited as long as it can send gaseous carbon dioxide, and, for example, a rotary pump may be employed.

The intermediate tank 15 temporarily stores carbon dioxide sent out from the pump 14. The pressure in the intermediate tank 15 may be, for example, 1 bar of an ordinary pressure.

The compressor 16 compresses gaseous carbon dioxide to liquidize. Then, the compressor 16 sends out liquidized carbon dioxide in order to use it in the CCUS cycle. The pressure of carbon dioxide liquidized by the compressor 16 may be, for example, a pressure equivalent to or higher than the liquidizing pressure.

Fig. 3(c) is a diagram illustrating the operation of the DAC system S2.

Fig. 3(c) indicates that the DAC system S2 is operated by conducting CO₂ adsorption, replacement, CO₂ desorption, and cooling.

In the step of "CO₂ adsorption", the blower 11 is operated to send out the air to the CO₂ adsorber 12. This brings carbon dioxide in the air into contact with the CO₂ adsorption layer 122 of the CO₂ adsorber 12. Then, carbon dioxide is adsorbed by the CO₂ adsorbent 122a of the CO₂ adsorption layer 122. It is noted that this step can be considered as an adsorption step of carbon dioxide. Then, the air transmitted through the CO₂ adsorber 12 is released into the air. In this step, the temperature of the air at which the air is sent out to the CO₂ adsorber 12 is, for example, 25°C, and the humidity is, for example, 40% RH. The concentration of carbon dioxide in the air is, for example, 400 ppm. The temperature of the air transmitted through the CO₂ adsorber 12 is, for example, 25°C, and the pressure is 1 bar of an ordinary pressure. Then, the CO₂ adsorption process is carried out, for example, for 10 minutes or longer, preferably for 1 hour, in the CO₂ adsorber 12.

In the step of "replacement", the pressure in the CO₂ adsorber 12 is reduced. Otherwise, a method for introducing gas, such as carbon dioxide or water vapor, may be employed. This can replace the air remaining in the CO₂ adsorber 12 after the CO₂ adsorption process, thereby increasing the CO₂ concentration at the desorption of CO₂. However, the replacement process may be omitted depending on the DAC system S2 in some cases. It is noted that this step can be considered as a replacement step for carbon dioxide. At this time, the replacement is carried out, for example, at a temperature of 25°C and a pressure of 1 bar or less in the CO₂ adsorber 12.

At this time, "CO₂ desorption" may be conducted. In the step of "CO₂ desorption", the pressure in the CO₂ adsorber 12 is reduced, and the CO₂ adsorber 12 is heated. Alternatively, only pressure reduction or only heating may be performed in some cases. It is noted that this step can be considered as a desorption step of carbon dioxide. At this time, the replacement is carried out, for example, at a temperature of 120°C or less and a pressure of 1 bar in the CO₂ adsorber 12.

As such, carbon dioxide adsorbed by the CO₂ adsorber 12 is desorbed by heating, pressure reduction, temperature variation, or the like and returns to the gaseous state.

Furthermore, in the step of "cooling", the CO₂ adsorber 12 is cooled. This makes the CO₂ adsorber 12 ready for adsorbing carbon dioxide again. It is noted that this step can be considered as a cooling step. At this time, the temperature of the CO₂ adsorber 12 reaches 25°C, and the pressure thereof reaches 1 bar of an ordinary pressure. Then, cooling is carried out, for example, for 10 minutes or longer in the CO₂ adsorber 12. Alternatively, the cooling step may be combined with the adsorption step in some cases.

This returns the CO₂ adsorber 12 to a state that can adsorb carbon dioxide again. That is, "CO₂ adsorption" can be performed again. The above process can then be repeated to capture carbon dioxide in the DAC system S2.

However, the CO₂ adsorbent 122a has a life span. If the CO₂ adsorbent 122a reaches the end of the life span thereof, it is necessary to regenerate the CO₂ adsorber 12. Regenerating the CO₂ adsorber 12 can save the cost required for the capture of carbon dioxide. However, in the CO₂ adsorber 12, the life span of the CO₂ adsorbent 122a is shorter than that of the substrate 121. Accordingly, when the CO₂ adsorber 12 is regenerated, the substrate 121 is reused as it is, and a new CO₂ adsorption layer 122 containing the CO₂ adsorbent 122a is formed. It is noted that this step can be considered as a regeneration step of the CO₂ adsorber 12.

When a new CO₂ adsorption layer 122 is formed, either a new CO₂ adsorption layer 122 is formed after removing the used CO₂ adsorption layer 122 that has reached the end of the life span thereof, or a new CO₂ adsorption layer 122 is formed without removing the used CO₂ adsorption layer 122. The former is a method for re-forming a new CO₂ adsorption layer 122 on the substrate 121 after removing a used old CO₂ adsorption layer 122. The latter is a method for re-forming a new CO₂ adsorption layer 122 on the used old CO₂ adsorption layer 122 by face coating. It is noted that the step of removing the CO₂ adsorption layer 122 that has adsorbed carbon dioxide can be considered as a removal step of the CO₂ adsorption layer 122. The step of forming a new CO₂ adsorption layer 122 on the substrate 121 can be considered as a re-forming step of the CO₂ adsorption layer 122.

To remove the used CO₂ adsorption layer 122 that has reached the end of the life span thereof, methods such as burning, acid dissolution, and alkali dissolution can be used.

In burning, the CO₂ adsorber 12 is heated to burn and remove the CO₂ adsorption layer 122. The lower limit of the heating temperature is, for example, 400°C or higher and preferably 500°C or higher, and the upper limit thereof is 700°C or lower and preferably 650°C or lower. The heating time is not limited as long as the CO₂ adsorption layer 122 can be removed and is, for example, 1 hour or longer and 48 hours or shorter.

In acid dissolution, an acidic solution is circulated through the CO₂ adsorber 12, and the CO₂ adsorption layer 122 is dissolved in the acidic solution to remove the CO₂ adsorption layer 122. Examples of acidic solutions may include aqueous solutions of hydrofluoric acid, sulfuric acid, nitric acid, hydrochloric acid, and phosphoric acid.

In alkali dissolution, an alkaline solution is circulated through the CO₂ adsorber 12, and the CO₂ adsorption layer 122 is dissolved in the alkaline solution to remove the CO₂ adsorption layer 122. Examples of alkaline solutions may include aqueous solutions of sodium hydroxide, potassium hydroxide, or the like.

To re-form a new CO₂ adsorption layer 122, the CO₂ adsorbent 122a and the carrier 122b are first put into a dispersion medium and stirred. This makes it possible to prepare a coating solution containing the CO₂ adsorbent 122a and the carrier 122b dispersed in the dispersion medium. Examples of dispersion media may typically include polar solvents such as water, alcohols, diols, NMP (N-methyl-2-pyrrolidone), and DMSO (dimethyl sulfoxide). Then, this coating solution is coated and dried. If necessary, the coating solution is further sintered. This makes it possible to form a new CO₂ adsorption layer 122.

### <Explanation on Management Apparatus 30>

Next, the management apparatus 30 will be described in detail.

Fig. 4 is a block diagram illustrating the functional configuration of the management apparatus 30.

The management apparatus 30 illustrated in Fig. 4 includes a concentration acquisition unit 31 that acquires the concentration of carbon dioxide; a captured amount calculation unit 32 that calculates the concentration of carbon dioxide; a management unit 33 that manages the CO₂ adsorber 12; a storage unit 34 that stores data relating to the CO₂ adsorber 12; and a notification unit 35 that notifies terminal apparatuses 20 and 21.

The concentration acquisition unit 31 acquires, from the CO₂ adsorber 12, a first concentration, which is a concentration of carbon dioxide on the inlet side of the CO₂ adsorber 12, and a second concentration, which is a concentration of carbon dioxide on the outlet side.

The captured amount calculation unit 32 calculates the captured amount of carbon dioxide adsorbed by the CO₂ adsorber 12 on the basis of the first concentration and the second concentration.

In this case, the captured amount calculation unit 32 first calculates the captured amount of carbon dioxide adsorbed by the CO₂ adsorber 12 by the following expression (1). (Captured ratio) = [(Inlet CO2 concentration) - (Outlet CO2 concentration)]/(Inlet CO2 concentration) ...

Then, the captured amount calculation unit 32 calculates a captured amount in one hour by the following expression (2). (Captured amount in one hour) = (Flow rate in one hour)/22.4 (L/mol) × 44 (g/mol) × (Inlet CO2 concentration) × (Captured ratio) ...

The flow rate in one hour can be calculated by the expression: (Flow velocity) × (Cross-sectional area of the CO₂ adsorber 12). 22.4 (L/mol) is the volume of the standard state of gases, and 44 (g/mol) is the molecular weight of carbon dioxide.

The captured amount in one day can be calculated as the accumulation of the captured amount in one hour.

As such, the captured amount calculation unit 32 calculates the captured amount on the basis of the first concentration and a second concentration, and the flow velocity of carbon dioxide.

At this time, the captured amount calculation unit 32 may calculate the captured amount by considering at least one of the temperature, the humidity, and the gas pressure of carbon dioxide. In other words, air is compensated for changes in the volume thereof due to temperature and gas pressure. In addition, because humidity changes the adsorption capacity of carbon dioxide on the CO₂ adsorber 12, the adsorption capacity is compensated for.

The first concentration and the second concentration acquired from the concentration acquisition unit 31, and the captured amount calculated by the captured amount calculation unit 32 are stored in the storage unit 34.

Fig. 5 is a diagram illustrating a data structure when these pieces of data are stored in the storage unit 34.

Fig. 5 shows a case where the concentration acquisition unit 31 stores the data acquired from the CO₂ adsorber 12 as Table T1.

The table T1 illustrated is created for each of a plurality of CO₂ adsorbers 12. In Fig. 5, Table T1 is created about three CO₂ adsorbers 12 represented as an adsorber A, an adsorber B, and an adsorber C.

Table T1 consists of data on flow velocity (Air flow velocity), inlet CO₂ concentration (CO₂ conc. Inlet), outlet CO₂ concentration (CO₂ conc. Outlet), captured ratio (Capture ratio), temperature (Temp), relative humidity (Humidity), gas pressure (Air Pressure), captured amount in one hour (Captured CO₂ in one hour), and captured amount in one day (Captured CO₂ in one day) with respect to the time (Time) at which various data is acquired.

Flow velocity is the flow velocity of air sent to the CO₂ adsorber 12. In addition, the inlet CO₂ concentration is the first concentration, which is the CO₂ concentration on the inlet side of the CO₂ adsorber 12. In addition, the outlet CO₂ concentration is the second concentration, which is the CO₂ concentration on the outlet side of the CO₂ adsorber 12.

A captured ratio represents a proportion of CO₂ that could be adsorbed by the CO₂ adsorber 12 with respect to carbon dioxide contained in the air. Furthermore, temperature, relative humidity, and gas pressure are the temperature, the humidity, the relative humidity, and the pressure, respectively, of the air sent to the CO₂ adsorber 12. The captured amount in one hour and the captured amount in one day are a captured amount per hour and a captured amount per day, respectively.

Return to Fig. 4, the management unit 33 conducts management to replace and regenerate the CO₂ adsorber 12 on the basis of a regeneration timing of the CO₂ adsorber 12 determined on the basis of the captured amount calculated by the captured amount calculation unit 32.

Fig. 6 is a conceptual diagram illustrating the management method of a CO₂ adsorber 12.

In this embodiment, the management unit 33 determines the regeneration timing of a plurality of CO₂ adsorbers 12 on the basis of the captured amount calculated by the captured amount calculation unit 32. Then, the management unit 33 stores the regeneration timing in the "Data base" of each CO₂ adsorber 12 in Fig. 6. This "Data base" corresponds to the storage unit 34 of the management apparatus 30.

Fig. 7 is a diagram illustrating the regeneration timing of a CO₂ adsorber 12.

In Fig. 7, the abscissa axis indicates time (month), and the ordinate axis indicates the average captured ratio in one month.

As illustrated, the average captured ratio of CO₂ adsorber 12 decreases over time due to the aging of the CO₂ adsorbent 122a. Then, the actual average captured ratio is shown by the solid polyline (ACHIEVEMENTS). In addition, the average captured ratio predicted from now on is shown by the dotted polyline (PREDICTED). Then, the average captured ratio of 75% is set as a regeneration execution threshold, and the time at which the actual average captured ratio falls below the regeneration execution threshold is set to a timing at which the replacement of the CO₂ adsorber 12 is essential. The area in which the average captured ratio is 75% or more and 85% or less is set to an alarm range, and the time at which an actual average captured ratio is in the alarm range is set to a timing at which the CO₂ adsorber 12 is recommended to be replaced.

Accordingly, the time at which an actual average captured ratio falls below the regeneration execution threshold is regarded as "immediately" as the regeneration timing. The time at which an actual average captured ratio is in the alarm range, the regeneration timing is considered as a timing at which, although there is no need to replace the CO₂ adsorber 12 immediately, the replacement work may be done depending on efficiency because the end of the life span is near.

Return to Fig. 6, the management unit 33 manages a plurality of CO₂ adsorbers 12 on the basis of the regeneration timing. In this embodiment, the CO₂ capturer enters into a contract with a CO₂ adsorber regenerator, such as a business operator who regenerates the CO₂ adsorber 12, or the like. In this case, the CO₂ adsorber regenerator replaces the CO₂ adsorber 12, manages the regeneration timing, and updates a CO₂ adsorbent 122a with the latest ability to the CO₂ capturer. The CO₂ capturer pays a regeneration fee to the CO₂ adsorber regenerator periodically or on case-by-case basis.

Then, according to this contract, the management unit 33 manages the CO₂ adsorber 12. Specifically, the management unit 33 manages the CO₂ adsorber 12 to replace each CO₂ adsorber 12 on the basis of the determined regeneration timing. When CO₂ adsorber 12 is replaced, the management unit 33 manages the regeneration history of the CO₂ adsorber 12. The regeneration history of the CO₂ adsorber 12 is stored in the storage unit 34 corresponding to the "Data base". The regeneration history of the CO₂ adsorber 12 is, for example, managed in the data structure indicated in the following Table T2.

Fig. 8 is a diagram illustrating Table T2 representing the regeneration history of a CO₂ adsorber 12.

Table T2 manages the models, Lot numbers, and whether the replacement is done or not of the substrate 121 and the CO₂ adsorbent 122a with respect to the time (Time) at which the replacement of the adsorber A, which is a CO₂ adsorber 12. That is, the management unit 33 manages the history of regeneration of each of the substrate 121 and the CO₂ adsorbent 122a.

"MODEL" indicates whether the substrate 121 and the CO₂ adsorbent 122a are new or old. Then, the management unit 13 manages the model defined by the type of the substrate 121 and the CO₂ adsorbent 122a. A CO₂ adsorbent 122a to be used in the regeneration of the CO₂ adsorber 12 is determined on the basis of this model. That is, when a newer type of the CO₂ adsorbent 122a than the CO₂ adsorbent 122a that has been used up until now is present at the regeneration of the CO₂ adsorber 12, a newer type CO₂ adsorbent 122a is used for regeneration.

"REPLACED ?" indicates whether the CO₂ adsorbent 122a was replaced with a new one at the regeneration. In this case, "Yes" means that the CO₂ adsorbent 122a was replaced with a new one, and "No" means that the CO₂ adsorbent 122a was not replaced and is used as it is.

The management unit 33 may further conduct the management of the CO₂ concentration sensors 13A and 13B. In this case, the management is a management of the calibration of the CO₂ concentration sensors 13A and 13B. If an error occurs in the measured value of the CO₂ concentration sensors 13A and 13B, an error also occurs in the captured amount of carbon dioxide calculated based thereon. If carbon dioxide emissions trading is to be based on this captured amount, the captured amount must be accurate. Accordingly, to ensure the accuracy of the CO₂ concentration sensors 13A and 13B, the CO₂ concentration sensors 13A and 13B need to be calibrated. Therefore, the management unit 33 also manages the CO₂ concentration sensors 13A and 13B and issues calibration certificates to guarantee measurement accuracy. Specifically, the calibration timing of the CO₂ concentration sensors 13A and 13B is managed so as to set the timing of calibration for every predetermined period (for example, every year).

The notification unit 35 notifies the CO₂ adsorber regenerator who regenerates the CO₂ adsorber 12 of regeneration timing. Actually, the notification unit 35 sends a notification to the terminal apparatus 21 (see Fig. 2) of the CO₂ adsorber regenerator, and the CO₂ adsorber regenerator receives this notification by the terminal apparatus 21.

In this case, as illustrated in Fig. 7, when the actual average captured ratio falls below the regeneration execution threshold, the notification unit 35 notifies that it is time at which the replacement of the CO₂ adsorber 12 is essential. When the actual average captured ratio is in the alarm range, the notification unit notifies that it is time at which the replacement of the CO₂ adsorber 12 is recommended. At this time, a timing at which the replacement of the CO₂ adsorber 12 is essential on the basis of the predicted average captured ratio, as illustrated in Fig. 7, may be notified.

As illustrated in Fig. 6, the management apparatus 30 allows a CO₂ capturer who captures carbon dioxide to monitor the captured amount of CO₂. This is performed by the notification unit 35 notifying the CO₂ capturer of the captured amount. For example, the captured amount is notified as a periodic report. Actually, the notification unit 35 sends a periodic report to a terminal apparatus 20 (see Fig. 2) of a CO₂ capturer, and the CO₂ capturer receives this periodic report at the terminal apparatus 20.

Figs. 9(a) and 9(b) are diagrams illustrating examples of periodic reports.

Among these, in Fig. 9(a), the abscissa axis indicates time, and the ordinate axis indicates the CO₂ captured amount in one hour and the average CO₂ captured ratio in one hour. Fig. 9(a) shows variations in the captured amount and captured ratio in one day. In Fig. 9(b), the abscissa axis indicates time, and the ordinate axis indicates the CO₂ captured amount in one day. Fig. 9(b) illustrates a cumulative value of the captured amount in one day.

### <Explanation of CO₂ Adsorption System 1>

Fig. 10 is a flowchart illustrating the operation of the CO₂ adsorption system 1.

First, the CO₂ adsorber 12 outputs the results of the CO₂ concentration sensors 13A and 13B (inlet/outlet sensors) (step S101). That is, the CO₂ adsorber 12 outputs a first concentration, which is the concentration of carbon dioxide on the inlet side of the CO₂ adsorber 12, and a second concentration, which is the concentration of carbon dioxide on the outlet side. Output results include data of time, flow velocity, temperature, relative humidity, and gas pressure, which are listed in Table T1 in Fig. 5.

Next, the concentration acquisition unit 31 of the management apparatus 30 acquires the output results from the CO₂ adsorber 12 and stores the output results in the storage unit 34 (step S102). The output results are stored in the format as in Table T1 of Fig. 5.

Then, the captured amount calculation unit 32 of the management apparatus 30 removes the noise components in the output results by a moving average method or the like (step S103). This removes noise components in the data of the first concentration and the second concentration.

Then, the captured amount calculation unit 32 of the management apparatus 30 calculates the captured ratio of carbon dioxide. The management unit 33 of the management apparatus 30 creates a periodic report, and the notification unit 35 sends a periodic report to a CO₂ capturer (step S104).

Then, the captured amount calculation unit 32 of the management apparatus 30 calculates the captured ratio, and the management unit 33 judges whether this captured ratio is equal to or larger than the threshold (step S105). This threshold is the regeneration execution threshold explained in Fig. 7.

As a result, if the captured ratio is equal to or larger than the threshold (YES in step S105), the CO₂ adsorber 12 is used as it is (step S106).

On the contrary, if the captured ratio is smaller than the threshold (NO in step S105), the management unit 33 judges that it is the regeneration timing of the CO₂ adsorber 12, and the notification unit 35 notifies the CO₂ adsorber regenerator of an alert (step S107). That is, the notification unit 35 notifies the CO₂ adsorber regenerator of regeneration timing.

The CO₂ adsorber regenerator replaces the CO₂ adsorber 12 and regenerates a used CO₂ adsorber 12. At this time, the management unit 33 stores the regeneration history of the CO₂ adsorber 12 in the format as in Table T2 of Fig. 8. The CO₂ adsorber regenerator determines whether the CO₂ adsorbent 122a is the newest or not on the basis of the regeneration history (step S108).

As a result, if it is the newest (YES in step S108), the CO₂ adsorber regenerator performs regeneration work of the CO₂ adsorber 12 using a current CO₂ adsorbent 122a (step S109).

In contrast, if it is not the newest (NO in step S108), the CO₂ adsorber regenerator conducts regeneration work of the CO₂ adsorber 12 using the newest CO₂ adsorbent 122a (step S110).

Then, the CO₂ adsorber regenerator inputs the regeneration history stored in the management apparatus 30 after conducting the regeneration work of the CO₂ adsorber 12 (step S111). Actually, the CO₂ adsorber regenerator inputs the contents of the regeneration work by the terminal apparatus 21 (see Fig. 2). The input data is sent to the management apparatus 30, and the management apparatus 30 stores the data as a regeneration history in the format, such as Table T2 in Fig. 8.

### <Explanation on Effect>

According to the mode described above, each CO₂ adsorber 12 can be replaced at an appropriate timing by managing the CO₂ adsorber 12, so-called, on time. Therefore, the management of the CO₂ adsorber 12 is easy, which saves cost.

According to the mode described above, a plurality of CO₂ capturers pay reuse fees to a CO₂ adsorber regenerator periodically or on case-by-case basis to replace, take back, and manage the CO₂ adsorber 12. By being entrusted with many projects, the CO₂ adsorber regenerator can respond efficiently and provide low-cost replacement and management of CO₂ adsorbers 12.

Furthermore, by having a plurality of CO₂ capturers pay reuse fees to a CO₂ adsorber regenerator periodically or on case-by-case basis, the CO₂ adsorber 12 is always updated to the newest CO₂ adsorbent 122a when it is replaced. The CO₂ adsorber regenerator can also provide a CO₂ adsorbent 122a at a low cost by being able to apply the newest CO₂ adsorbent 122a to many projects. At this time, a systematic regeneration plan for the CO₂ adsorber 12 can be created on the basis of the predicted average captured ratio shown in Fig. 7. Therefore, the stock amount of CO₂ adsorbents 122a held by the CO₂ adsorbent regenerator can be reduced, and even when the CO₂ adsorbent 122a is switched to a new type, the occurrence of dead stock of the old type of the CO₂ adsorbent 122a can be controlled.

In addition, by having a plurality of CO₂ collectors pay fees to the CO₂ adsorber regenerator periodically or on case-by-case basis, it is also possible to outsource the management and calibration work of the surrounding measurement apparatuses, including the CO₂ concentration sensors 13A and 13B. Since the CO₂ adsorber regenerator can be commissioned to handle many projects and can handle the projects at an efficient frequency on the basis of the achievements stored in the management apparatus 30, the replacement and management of the CO₂ adsorbs 12 can be provided at a low cost. Examples of surrounding measurement apparatuses other than the CO₂ concentration sensors 13A and 13B may include air velocity meters that measure flow velocity, thermometers for the measurement of surrounding environments, hygrometers, barometers, and the like.

When the CO₂ concentration sensors 13A and 13B are managed, the CO₂ concentration sensors 13A and 13B are calibrated at predetermined intervals, and issuance of a calibration certificate can guarantee the captured amount of carbon dioxide.

Examples of acidic gases may include, in addition to carbon dioxide (CO₂), hydrogen sulfide (H₂S); sulfur oxide (SOx) such as sulfur dioxide (SO₂); nitrogen dioxide (NO₂); dimethyl sulfide (DMS); and chloride hydrogen (HCl).

In the embodiment described above, a case where carbon dioxide in the air is captured by adsorption has been described, but embodiments are not limited thereto. For example, coke oven gas emitted from a coke oven contains hydrogen sulfide. When the coke oven gas is used as fuel, sulfur oxides are emitted if the coke oven gas contains hydrogen sulfide. Since sulfur oxides are harmful, it is sometimes desirable to remove (desulfurize) hydrogen sulfide in advance and avoid the generation of sulfur oxides. The mode described above can be applied to a case where hydrogen sulfide in the coke oven gas is removed. In this case, the gas is coke oven gas, and the acidic gas is hydrogen sulfide. If fossil fuel is burned, sulfur oxide is released with carbon dioxide. At this time, it may be desirable to remove (desulfurize) sulfur oxides with carbon dioxide in some cases. Accordingly, the mode described above can be applied to the case where it is desirable to remove sulfur oxides. In this case, the gas is air, and the acidic gas is carbon dioxide or sulfur oxide.

### <Explanation on Management Method>

Here, the process conducted by the management apparatus 30 can be considered as a management method including: acquiring a first concentration, which is a concentration of an acidic gas when a gas containing the acidic gas is introduced into an acidic gas adsorber that adsorbs the acidic gas, and a second concentration, which is a concentration of the acidic gas after being processed in the acidic gas adsorber; calculating a captured amount of the acidic gas adsorbed by the acidic gas adsorber on the basis of the first concentration and the second concentration; and conducting management to replace and regenerate the acidic gas adsorber on the basis of a regeneration timing of the acidic gas adsorber determined on the basis of the calculated captured amount.

As stated above, this embodiment has been described, but the technical scope of the present invention is not limited to the scope disclosed in the above embodiment. It is also clear from the recitations of claims that various variations or modifications of the above embodiment are also encompassed in the technical scope of the present invention. For example, items in the various data illustrated in Fig. 5, the average captured ratio in one month of carbon dioxide illustrated in Fig. 7, the regeneration history of the CO₂ adsorber 12 illustrated in Fig. 8, and the periodic report illustrated in Fig. 9 are exemplary, and the items and values listed in these figures may be other items and other values.

### [Reference Signs List]

- 1: CO₂ adsorption system
- 12: CO₂ adsorber
- 13A, 13B: CO₂ concentration sensor
- 20, 20A, 20B, 20C, 21: Terminal apparatus
- 30: Management apparatus
- 31: Concentration acquisition unit
- 32: Captured amount calculation unit
- 33: Management unit
- 34: Storage unit
- 35: Notification unit
- 121: Substrate
- 122: CO₂ adsorption layer
- 122a: CO₂ adsorbent
- 122b: Carrier
- S: CCUS cycle
- S2: DAC system
- K1, K2, K3: CO₂ capturer
- R1: CO₂ adsorber regenerator

## Claims

1. A management apparatus comprising:
a concentration acquisition unit configured to acquire a first concentration which is a concentration of an acidic gas when a gas containing the acidic gas is introduced into an acidic gas adsorber that adsorbs the acidic gas and a second concentration which is a concentration of the acidic gas after being processed in the acidic gas adsorber;
a captured amount calculation unit configured to calculate a captured amount of the acidic gas adsorbed by the acidic gas adsorber based on the first concentration and the second concentration; and
a management unit configured to conduct management to replace and regenerate the acidic gas adsorber based on a regeneration timing of the acidic gas adsorber determined based on the calculated captured amount.

2. The management apparatus according to claim 1, wherein the acidic gas adsorber comprises a substrate and an acidic gas adsorption layer that includes an acidic gas adsorbent formed on a surface of the substrate and configured to adsorb the acidic gas, and
when regenerating the acidic gas adsorber, the management apparatus forms a new acidic gas adsorption layer.

3. The management apparatus according to claim 2, wherein the new acidic gas adsorption layer is formed by either forming the new acidic gas adsorption layer after removing the used acidic gas adsorption layer or forming the new acidic gas adsorption layer without removing the used acidic gas adsorption layer.

4. The management apparatus according to claim 2, wherein the management unit manages a model determined depending on a type of the acidic gas adsorbent and determines the acidic gas adsorbent to be used depending on the model at the regeneration of the acidic gas adsorber.

5. The management apparatus according to claim 4, wherein the model indicates whether the acid gas adsorbent is new or old.

6. The management apparatus according to claim 2, wherein the management unit manages a regeneration history for each of the substrate and the acidic gas adsorbent.

7. The management apparatus according to claim 1, further comprising a notification unit configured to notify an acidic gas adsorber regenerator who regenerates the acidic gas adsorber of the regeneration timing.

8. The management apparatus according to claim 7, wherein the regeneration timing includes a timing at which the replacement of the acidic gas adsorber is essential and a timing at which the acidic gas adsorber is recommended to be replaced.

9. The management apparatus according to claim 7, wherein the notification unit notifies an acidic gas capturer who captures the acidic gas of the captured amount.

10. The management apparatus according to claim 1, wherein the captured amount is calculated based on the first concentration and the second concentration, and a flow velocity of the acidic gas.

11. The management apparatus according to claim 10, wherein the captured amount is calculated by considering at least one of temperature, humidity, and gas pressure of the acidic gas.

12. The management apparatus according to claim 1, wherein the management unit manages concentration measurement means configured to measure the first concentration and the second concentration.

13. The management apparatus according to claim 12, wherein the management is a management of calibration of the concentration measurement means.

14. A management method comprising:
acquiring a first concentration which is a concentration of an acidic gas when a gas containing the acidic gas is introduced into an acidic gas adsorber that adsorbs the acidic gas and a second concentration which is a concentration of the acidic gas after being processed in the acidic gas adsorber;
calculating a captured amount of the acidic gas adsorbed by the acidic gas adsorber based on the first concentration and the second concentration; and
conducting management to replace and regenerate the acidic gas adsorber based on a regeneration timing of the acidic gas adsorber determined based on the calculated captured amount.

15. An acidic gas adsorption system comprising:
an acidic gas adsorber configured to adsorb an acidic gas;
concentration measurement means configured to measure a first concentration which is a concentration of the acidic gas when the acidic gas is introduced into the acidic gas adsorber and a second concentration which is a concentration of the acidic gas after being processed in the acidic gas adsorber, and
a management apparatus configured to manage the acidic gas adsorber based on the first concentration and the second concentration;
the management apparatus including:
a concentration acquisition unit configured to acquire the first concentration and the second concentration from the concentration measurement means;
a captured amount calculation unit configured to calculate a captured amount of the acidic gas adsorbed by the acidic gas adsorber based on the first concentration and the second concentration; and
a management unit configured to conduct management to replace and regenerate the acidic gas adsorber based on a regeneration timing of the acidic gas adsorber determined based on the calculated captured amount.
